# FASCICULE DE BREVET EUROPEEN

(11) **EP 1 348 018 B1**
(45) Date de publication et mention de la délivrance du brevet: **14.07.2010**
(21) Numéro de dépôt: 02710944.6
(22) Date de dépôt: 04.01.2002
(51) Int. Cl.: C12N 1/20, C12Q 1/04

(54) **MILIEU DE CULTURE POUR LA DETECTION DES BACTERIES DU GENRE LISTERIA**
KULTURMEDIUM ZUM NACHWEIS VON BAKTERIEN DER GATTUNG LISTERIA
CULTURE MEDIUM FOR DETECTING BACTERIA OF LISTERIA GENUS

(30) Priorité: 05.01.2001 FR 0100121
(43) Date de publication de la demande: 01.10.2003
(73) Titulaire: Rambach, Alain, F-75006 Paris (FR)
(72) Inventeur: Rambach, Alain, F-75006 Paris (FR)
(74) Mandataire: Ahner, Francis
(86) Numéro de dépôt international: PCT/FR2002/000024
(87) Numéro de publication internationale: WO 2002/053705

(56) Documents cités:
- US-A- 5 330 889
- US-A- 5 962 251
- KERR K G ET AL: "EVALUATION OF THE ROSCO SYSTEM FOR THE IDENTIFICATION OF LISTERIA-SPP" JOURNAL OF MEDICAL MICROBIOLOGY, vol. 35, no. 4, 1991, pages 193-196, XP001035023 ISSN: 0022-2615
- CARLSON P ET AL: "EVALUATION OF A COMMERCIAL KIT IN THE IDENTIFICATION OF ARCANOBACTERIUM HAEMOLYTICUM AND ACTINOMYCES PYOGENES" EUROPEAN JOURNAL OF CLINICAL MICROBIOLOGY & INFECTIOUS DISEASES, SPRINGER, WIESBADEN, DE, vol. 13, no. 6, 1994, pages 507-509, XP001030477 ISSN: 0934-9723
- CORRAL DEL F ET AL: "EVALUATION OF THE API-ZYM SYSTEM FOR IDENTIFICATION OF LISTERIA" FOOD MICROBIOLOGY, ACADEMIC PRESS LTD, LONDON, GB, vol. 7, no. 2, 1990, pages 99-106, XP001028505 ISSN: 0740-0020
- WALKLEY S U ET AL: "Bone marrow transplantation corrects the enzyme defect in neurons of the central nervous system in a lysosomal storage disease." PROCEEDINGS OF THE NATIONAL ACADEMY OF SCIENCES OF THE UNITED STATES, vol. 91, no. 8, 1994, pages 2970-2974, XP002184506 1994 ISSN: 0027-8424
- RESTAINO L. ET AL: 'Isolation and detection of Listeria monocytogenes using fluorogenic and chromogenic substrates for phosphatidylinositol-specific phospholipase C' JOURNAL OF FOOD PROTECTION vol. 62, no. 3, 1999, pages 244 - 251, XP009020877
- PETER KÄMPFER ET AL: 'Glycosidase profiles of members of the family Enterobacteriaceae' JOURNAL OF CLINICAL MICROBIOLOGY vol. 29, no. 12, Décembre 1991, pages 2877 - 2879, XP000937655
- PETER KÄMPFER: 'Differentiation of Corynebacterium spp., Listeria spp., and related organisms by using fluorogenic substrates' JOURNAL OF CLINICAL MICROBIOLOGY vol. 30, no. 5, Mai 1992, pages 1067 - 1071, XP000937654

## Description

La présente invention concerne un milieu de culture chromogène destiné à mettre en évidence les bactéries du genre *Listeria,* notamment *Listeria monocytogenes,* ledit milieu étant un milieu de culture solide pour la détection et/ou la discrimination de bactéries du genre *Listeria,* **caractérisé en ce qu**'il comporte, dans un milieu de culture de *Listeria,* au moins un agent chromogène choisi parmi les substrats de l'α-mannosidase, et en ce que ledit agent chromogène libère par hydrolyse un chromophore précipitable choisi parmi les dérivés indoxyle, halogéno-indoxyle (bromo-indoxyle, chloro-indoxyle, fluoro-indoxyle, iodo-indoxyle, dichloro-indoxyle, chloro-bromo- indoxyle, tri-chloro-indoxyle), méthyl-indoxyle, ou hydroxy-quinoline.

Tant dans le domaine clinique que dans le domaine agro-alimentaire, la recherche de *Listeria,* et en particulier de *Listeria monocytogenes* est de plus en plus importante, car ces bactéries sont souvent disséminées dans des produits agro-alimentaires et conduisent à des infections graves chez les patients sensibles (femmes enceintes, personnes âgées, etc...).

De fait, depuis quelques années, les gouvernements mettent en place des réseaux de surveillance de plus en plus stricts, notamment pour les produits de l'industrie agro-alimentaire. Ainsi, on observe une généralisation de la surveillance épidémiologique de *Listeria monocytogenes*.

Il est donc important de disposer d'un test fiable et rapide permettant de détecter les contaminations par ces bactéries, lequel test devant être à la fois sensible et spécifique.

Il existe aujourd'hui certains milieux de culture pour la détection de *Listera*, tels le PALCAM ou le milieu OXFORD plus ou moins sélectifs qui permettent de détecter la présence de *Listeria* dans des échantillons. Les résultats obtenus en utilisant ces milieux peuvent présenter certaines imprécisions impliquant la nécessité d'effectuer d'autres tests pour confirmer la présence de *L. monocytogenes.*

Ces milieux reposent en effet sur une combinaison d'antimicrobiens apportant une sélectivité et la détection d'enzymes apportant une spécificité. Toutefois, à la fois la sélectivité et la spécificité de ces milieux peuvent être améliorées, car ils conduisent en particulier à la présence de nombreux faux positifs ou faux négatifs. Un désavantage de ces milieux est qu'ils ne permettent pas la distinction de l'espèce *L. monocytogenes.*

II existe aussi des milieux qui incluent un test reposant sur la détection de phospholipases pour détecter directement *L. monocytogenes* par une coloration spécifique de la colonie ou par un halo spécifique entourant la colonie. Ainsi, Restaino et al (J Food Prot. 1999 Mar;62(3):244-51) décrit un procédé de détection et/ou de discrimination des bactéries du genre *Listeria* qui comprend un milieu de culture de *Listeria* contenant un substrat chromogène et fluorogène spécifique d'identification de l'enzyme phosphatidylinositol phospholipase C (PI-PLC). Toutefois, ces tests sont aussi positifs pour *L. ivanovii,* qui présente également un caractère phospholipase +.

Il était suggéré de différentier *L. monocytogenes* de *L. ivanovii* dans une étape d'identification suivant l'étape d'isolement, en utilisant notamment un test basé sur une différence dans un phénotype « aminopeptidase ».

Ainsi, le brevet US 5,330,889 concerne un procédé et un milieu d'identification de bactéries du genre Listeria et en particulier *Listeria monocytogenes,* ledit milieu comprenant un substrat chromogène ou fluorogène capable d'être hydrolysé par une glycine aminopeptidase.

Par ailleurs, Kämpfer (J Clin Microbiol. 1992 May;30(5):1067-71) décrit l'utilisation d'un milieu liquide comprenant un substrat de l'a-mannosidase pour la détection de bactéries du genre Listeria. La composition spécifique du milieu utilisé est divulguée dans Kämpfer et al (J Clin Microbiol. 1991 Dec;29(12):2877-9), le substrat de l'a-mannosidase utilisé étant l'agent fluorogène 4-methylumbelliferyl-α**-**D-mannopyranoside, qui n'est pas précipitable. Au contraire, il est capable de diffuser à l'intérieur du milieu solide et n'est donc pas utilisable en milieu solide.

Kerr et al (J Med Microbiol. 1991 Oct;35(4):193-6) décrit également l'utilisation d'un milieu liquide comprenant un substrat de l'α-mannosidase pour la détection de bactéries du genre Listeria. Dans ce cas, le substrat de l'α-mannidase utilisé est le p-nitrophenyl-α-D-mannopyranoside, qui n'est pas non plus précipitable, mais est au contraire capable de diffuser à l'intérieur du milieu solide et n'est donc pas utilisable en milieu solide.

Aux fins de différentiation de *L. monocytogenes* de *L. ivanovii,* la présente invention se base sur la détection de l'alpha mannosidase, un caractère positif pour *L. monocytogenes* et négatif pour *L. ivanovii.* Ce caractère peut par exemple être révélé sur des cultures pures à l'aide du substrat enzymatique nitrophényl-α-mannoside, qui est incolore et qui libère un nitrophényl coloré en jaune lorsque le test est positif.

La présente invention démontre que l'on peut révéler l'α-mannosidase au niveau des colonies sur milieu solide en détectant l'α-mannosidase, avec un substrat chromogène dans le milieu solide. L'utilisation d'un substrat de l'α-mannosidase dans un milieu solide, décrite pour la première fois dans la présente demande, présente notamment l'avantage de préparer des milieux solides où l'on peut différentier dès l'isolement, sans avoir à effectuer de tests supplémentaires, L. *monocytogenes* de *L. ivanovii.*

Ainsi, la présente invention concerne un nouveau milieu de culture solide, pour la détection et/ou la discrimination de bactéries du genre *Listeria,* **caractérisé en ce qu'**il comporte, dans un milieu de culture de *Listeria,* au moins un un agent chromogène choisi parmi les substrats de l'α-mannosidase, et en ce que ledit agent chromogéne libère par hydrolyse

un chromophore précipitable choisi parmi les dérivés indoxyle, halogéno-indoxyle (bromo-indoxyle, chloro-indoxyle, fluoro-indoxyle, iodo- indoxyle, dichloro-indoxyle, chloro-bromo-indoxyle, tri-chloro-indoxyle), le méthyl-indoxyle, ou hydroxy-quinoline. Ainsi, la colonie bactérienne se colore en fonction du chromophore libéré, le chromophore libéré étant solide dans le milieu de culture, et restant donc localisé au niveau de la colonie où il a été libéré.

Des dérivés préférés sont choisis en particulier parmi les dérivés suivants : 6-chloro-indoxyle, 5-bromo-indoxyle, 3-bromo-indoxyle, 6-fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle et 8-hydroxy-quinoline.

De façon préférée, le substrat de l'-mannosidase est l'indoxyl-α-mannoside. La concentration de l'agent chromogène dans le milieu est comprise entre environ 0,01 et 0,5 g/l. Une concentration préférée est 0,05 g/l.

Il peut également être intéressant de rajouter un activateur de réaction colorée, dans le milieu, afin d'améliorer la qualité de la réaction observée. Un activateur approprié pour un tel usage est le méthyl-α-mannoside, qui est intégré au milieu à une concentration comprise entre environ 0,01 et 0,5 g/l, de préférence 0,25 g/l.

Le milieu selon la présente invention permet ainsi de détecter *L. monocytogenes,* et de discriminer cette bactérie par rapport à *L. ivanovii*, sans effectuer de test supplémentaire. Afin d'optimiser les résultats donnés par le milieu selon l'invention, il peut être intéressant de rajouter des facteurs sélectifs pour *Listeria,* ainsi qu'utilisée dans les milieux de l'art antérieur. On obtient donc un milieu spécifique pour *L. monocytogenes.*

L'invention a également pour objet l'utilisation d'un milieu de culture selon l'invention pour la détection et/ou la discrimination des bactéries du genre *Listeria,* notamment *L. monocytogenes.*

La présente invention a également pour objet un procédé de détection et/ou de discrimination des bactéries du genre *Listeria* dans un échantillon, **caractérisé en ce qu'**il comprend les étapes suivantes :
a. inoculer un milieu de culture selon l'invention avec ledit échantillon ou un inoculum dérivé de l'échantillon,
b. détecter la présence de bactéries du genre *Listeria* sur ledit milieu de culture,
c. de façon optionnelle, différentier *L. monocytogenes,* de *L. ivanovii* et des autres *Listeria* présentes sur ledit milieu de culture.

### EXEMPLES

### Exemple 1

Un milieu préféré pour la mise en oeuvre de l'invention comprend (pour un litre):
- Agar 10 g
- Infusion de cervelle de veau 12,5 g
- Protéose peptone 10 g
- Infusion de coeur de boeuf 5 g
- Chlorure de sodium 5 g
- Na₂HPO₄ 4 g
- KH₂PO₄ 2 g
- Glucose 2 g
- Chlorure de Lithium 7,5 g
- Lécithine 3 g
- Ofloxacine 0,0004 g
- Colistine 0,015 g
- Ceftazidime 0,0134 g
- Méthyl-α-mannoside 0,25 g
- Indoxyl-α-mannoside 0,05 g

### Exemple 2

L'étalement de bactéries le milieu selon l'invention a donné les résultats suivants (24 heures d'incubation à 37°C).

| | Halo | Centre des colonies |
|---|---|---|
| *L. monocytogenes* | blanc | bleu |
| *L. ivanovii* | blanc | incolore |
| Autres *Listeria* | pas de halo | ------ |

L'utilisation du milieu selon l'invention permet donc de détecter et discriminer *L. monocytogenes, L. ivanovii,* et les autres espèces de *Listeria.*

## Revendications

1. Milieu de culture solide pour la détection et/ou la discrimination de bactéries du genre *Listeria,* **caractérisé en ce qu'**il comporte, dans un milieu de culture de *Listeria,* au moins un agent chromogène choisi parmi les substrats de l'α-mannosidase, et **en ce que** ledit agent chromogène libère par hydrolyse un chromophore précipitable choisi parmi les dérivés indoxyle, halogéno-indoxyle (bromo-indoxyle, chloro-indoxyle, fluoro-indoxyle, iodo-indoxyle, dichloro-indoxyle, chloro-bromo- indoxyle, tri-chloro-indoxyle), méthyl-indoxyle, ou hydroxy-quinoline.

2. Milieu de culture selon la revendication 1, **caractérisé en ce que** ledit agent chromogène libère, par hydrolyse, un chromophore précipitable choisi parmi les dérivés 6-chloro-indoxyle, 5-bromo-indoxyle, 3-bromo-indoxyle, 6- fluoro-indoxyle, 5-iodo-indoxyle; 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle ou 8-hydroxy-quinoline.

3. Milieu de culture selon l'une des revendications 1 à 2, **caractérisé en ce que** ledit milieu contient également un activateur de réaction colorée.

4. Milieu de culture selon la revendication 3, **caractérisé en ce que** ledit activateur est le méthyl-α-mannoside.

5. Milieu de culture selon l'une des revendications 1 à 4, **caractérisé en ce qu'**il comporte en outre des facteurs sélectifs pour *Listeria.*

6. Milieu de culture selon l'une des revendications 1 à 5, **caractérisé en ce que** le substrat de l'α-mannosidase est l'indoxyl-α-mannoside.

7. Milieu de culture selon l'une des revendications 1 à 6, **caractérisé en ce qu'**il comprend (pour un litre) :
- Agar 10 g
- Infusion de cervelle de veau 12,5 g
- Protéose peptone 10 g
- Infusion de coeur de boeuf 5 g
- Chlorure de sodium 5 g
- Na₂HPO₄ 4 g
- KH₂PO₄ 2 g
- Glucose 2 g
- Chlorure de Lithium 7,5 g
- Lécithine 3 g
- Ofloxacine 0,0004 g
- Colistine 0,015 g
- Ceftazidime 0,0134 g
- Méthyl-α-mannoside 0,25 g
- Indoxyl-α-mannoside 0,05 g

8. Milieu de culture selon l'une des revendications 1 à 7, **caractérisé en ce qu'**il est spécifique de *L. monocytogenes.*

9. Utilisation d'un milieu de culture tel que défini dans l'une des revendications 1 à 8 pour la détection et/ou la discrimination des bactéries du genre *Listeria.*

10. Procédé de détection et/ou discrimination des bactéries du genre *Listeria* dans un échantillon, **caractérisé en ce qu'**il comprend les étapes consistant à :
a. inoculer un milieu de culture tel que défini dans l'une des revendications 1 à 8 avec ledit échantillon ou un inoculum dérivé de l'échantillon,
b. détecter la présence de bactéries du genre *Listeria* sur ledit milieu de culture,
c. de façon optionnelle, différentier *L. monocytogenes* de *L. ivanovii* et des autres *Listeria* présentes sur ledit milieu de culture.

## Claims

1. Solid culture medium for detecting and/or discriminating bacteria of the *Listeria* genus, **characterized in that** it comprises, in a *Listeria* culture medium, at least one chromogenic agent chosen from α-mannosidase substrates, and **in that** said chromogenic agent releases, by hydrolysis, a precipitatable chromophore chosen from the indoxyl, haloindoxyl (bromoindoxyl, chloroindoxyl, fluoroindoxyl, iodoindoxyl, dichloroindoxyl, chlorobromoindoxyl, trichloroindoxyl), methylindoxyl or hydroxyquinoline derivatives.

2. Culture medium according to Claim 1, **characterized in that** said chromogenic agent releases, by hydrolysis, a precipitatable chromophore chosen from the 6-chloroindoxyl, 5-bromoindoxyl, 3-bromoindoxyl, 6-fluoroindoxyl, 5-iodoindoxyl, 4,6-dichloroindoxyl, 6,7-dichloroindoxyl, 5-bromo-4-chloroindoxyl, 5-bromo-6-chloroindoxyl, 4,6,7-trichloroindoxyl, N-methylindoxyl or 8-hydroxyquinoline derivatives.

3. Culture medium according to either of Claims 1 and 2, **characterized in that** said medium also contains a colored-reaction activator.

4. Culture medium according to Claim 3, **characterized in that** said activator is methyl-α-mannoside.

5. Culture medium according to one of Claims 1 to 4, **characterized in that** it also comprises factors selective for *Listeria.*

6. Culture medium according to one of Claims 1 to 5, **characterized in that** the α-mannosidase substrate is indoxyl-α-mannoside.

7. Culture medium according to one of Claims 1 to 6, **characterized in that** it comprises (per liter):
- agar 10 g
- calf brain infusion 12.5 g
- proteose peptone 10 g
- beef heart infusion 5 g
- sodium chloride 5 g
- Na₂HPO₄ 4 g
- KH₂PO₄ 2 g
- glucose 2 g
- lithium chloride 7.5 g
- lecithin 3 g
- ofloxacin 0.0004 g
- colistin 0.015 g
- ceftazidime 0.0134 g
- methyl-α-mannoside 0.25 g
- indoxyl-α-mannoside 0.05 g

8. Culture medium according to one of Claims 1 to 7, **characterized in that** it is specific for *L. monocytogenes.*

9. Use of a culture medium as defined in one of Claims 1 to 8, for detecting and/or discriminating bacteria of the *Listeria* genus.

10. Method for detecting and/or discriminating bacteria of the *Listeria* genus in a sample, **characterized in that** it comprises the steps consisting in:
a. inoculating a culture medium as defined in one of Claims 1 to 8 with said sample or an inoculum derived from the sample,
b. detecting the presence of bacteria of the *Listeria* genus on said culture medium,
c. optionally, differentiating *L. monocytogenes* from *L. ivanovii* and from the other *Listeria* present on said culture medium.

## Patentansprüche

1. Festes Kulturmedium für den Nachweis und/oder die Unterscheidung von Bakterien der Gattung *Listeria,* **dadurch gekennzeichnet, dass** es in einem Listeria-Kulturmedium mindestens ein chromogenes Mittel umfasst, das aus Substraten von α-Mannosidase ausgewählt ist, und **dadurch**, dass das chromogene Mittel durch Hydrolyse einen fällbaren Chromophor freisetzt, der ausgewählt ist aus Indoxyl-, Halogenindoxyl-, (Bromindoxyl-, Chlorindoxyl-, Fluorindoxyl-, lodindoxyl-, Dichlorindoxyl-, Chlorbromindoxyl-, Trichlorindoxyl-), Methylindoxyl- oder Hydroxychinolin-Derivaten.

2. Kulturmedium nach Anspruch 1, **dadurch gekennzeichnet, dass** das chromogene Mittel durch Hydrolyse einen fällbaren Chromophor freisetzt, der aus 6-Chlorindoxyl-, 5-Bromindoxyl-, 3-Bromindoxyl-, 6-Fluorindoxyl-, 5-lodindoxyl-, 4,6-Dichlorindoxyl-, 6,7-Dichlorindoxyl-, 5-Brom-4-chlorindoxyl-, 5-Brom-6-chlorindoxyl-, 4,6,7-Trichlorindoxyl-, N-Methylindoxyl- oder 8-Hydroxychinolin-Derivaten ausgewählt ist.

3. Kulturmedium nach einem der Ansprüche 1 bis 2, **dadurch gekennzeichnet, dass** das Medium auch einen Aktivator für die Farbreaktion enthält.

4. Kulturmedium nach Anspruch 3, **dadurch gekennzeichnet, dass** der Aktivator Methyl-α-mannosid ist.

5. Kulturmedium nach einem der Ansprüche 1 bis 4, **dadurch gekennzeichnet, dass** es darüber hinaus selektive Faktoren für *Listeria* umfasst.

6. Kulturmedium nach einem der Ansprüche 1 bis 5, **dadurch gekennzeichnet, dass** das Substrat von α-Mannosidase Indoxyl-α-mannosid ist.

7. Kulturmedium nach einem der Ansprüche 1 bis 6, **dadurch gekennzeichnet, dass** es umfasst (pro Liter):
- Agar 10 g
- Kälberhirnaufguss 12,5
- Proteose-Pepton 10 g
- Rinderherzaufguss 5 g
- Natriumchlorid 5 g
- Na₂HPO₄ 4 g
- KH₂PO₄ 2 g
- Glucose 2 g
- Lithiumchlorid 7,5 g
- Lecithin 3 g
- Ofloxacin 0,0004 g
- Colistin 0,015 g
- Ceftazidim 0,0134 g
- Methyl-α-mannosid 0,25 g
- Indoxyl-α-mannosid 0,05 g

8. Kulturmedium nach einem der Ansprüche 1 bis 7, **dadurch gekennzeichnet, dass** es für *L. monocytogenes* spezifisch ist.

9. Verwendung eines Kulturmediums, wie in einem der Ansprüche 1 bis 8 definiert, für den Nachweis und/oder die Unterscheidung von Bakterien der Gattung *Listeria.*

10. Verfahren zum Nachweis und/oder zur Unterscheidung von Bakterien der Gattung *Listeria* in einer Probe, **dadurch gekennzeichnet, dass** es die Schritte umfasst, die bestehen aus:
a. Inokulieren eines Kulturmediums, wie in einem der Ansprüche 1 bis 8 definiert, mit der Probe oder einem Inokulum, das von der Probe abstammt,
b. Nachweis der Anwesenheit von Bakterien der Gattung *Listeria* auf dem Kulturmedium,
c. gegebenenfalls Unterscheiden von *L. monotytogenes von L. ivanovii* und anderen *Listeria,* die auf dem Kulturmedium anwesend sind.
